Europäisches Patentamt

European Patent Office

Office européen des brevets

(11) Veröffentlichungsnummer: **0 429 868 A2**

# (12) EUROPÄISCHE PATENTANMELDUNG

(21) Anmeldenummer: **90120738.1**

(22) Anmeldetag: **29.10.90**

(51) Int. Cl.5: **A61K 31/55, A61K 31/40**

Die Bezeichnung der Erfindung wurde geändert (Richtlinien für die Prüfung im EPA, A-III, 7.3).

(30) Priorität: 30.10.89 US 428555
30.10.89 US 428558
30.10.89 US 428559

(43) Veröffentlichungstag der Anmeldung:
05.06.91 Patentblatt 91/23

(84) Benannte Vertragsstaaten:
AT BE CH DE DK ES FR GB GR IT LI LU NL SE Patentblatt

(71) Anmelder: F. HOFFMANN-LA ROCHE AG
Postfach 3255
CH-4002 Basel(CH)·

(72) Erfinder: Hsu, Ming-Chu
445 East 86th Street, Apt. 15G
New York, NY 10028(US)
Erfinder: Tam, Steve Yik-Kai
13 Evergreen Road
West Caldwell, NJ 07006(US)

(74) Vertreter: Lederer, Franz, Dr. et al
Patentanwalt Dr. Franz Lederer
Lucile-Grahn-Strasse 22
W-8000 München 80(DE)

(54) **Verwendung eines Benzodiazepin- und eines Phenylpyrrylketon-Derivats bei retroviralen Infektionen.**

(57) Die Verbindungen 7-Chlor-5-(2-pyrryl)-3H-1,4-benzodiazepin-2(1H)-on, 2-Glycinamido-5-chlorphenyl-(2-pyrryl)keton und pharmazeutisch anwendbare Salze davon zeigen anti-retrovirale Aktivität und sind infolgedessen zur Behandlung und Prophylaxe von durch Retroviren verursachten Infektionserkrankungen geeignet.

EP 0 429 868 A2

# VERWENDUNG EINES BENZODIAZEPIN- UND EINES PHENYLPYRRYLKETON-DERIVATS

Die Erfindung beruht auf der Feststellung, dass die in der USP 3 405 122 beschriebene Verbindung 7-Chlor-5-(2-pyrryl)-3H-1-4-benzodiazepin-2(1H)-on (im folgenden Verbindung A genannt) und die in der USP 3 407 211 beschriebene Verbindung 2-Glycinamido-5-chlorphenyl-(2-pyrryl)keton (im folgenden Verbindung B genannt) und pharmazeutisch anwendbare Salze davon zur Behandlung und Prophylaxe von durch Retroviren verursachten Infektionskrankheiten geeignet sind, speziell zur Linderung der destruktiven cytopathischen Effekte retroviraler Erkrankungen, insbesondere solcher, die durch Retrovirus HIV, insbesondere HIV-1, verursacht sind.

Pharmazeutisch anwendbare Salze können Salze organischer Säuren, wie Milchsäure, Essigsäure, Aepfelsäure oder p-Toluolsulfonsäure sein oder von Mineralsäuren, wie Schwefelsäure oder Salzsäure.

Die Erfindung betrifft die Verwendung der oben genannten Verbindungen und Salze bei der Behandlung infektiöser Erkrankungen, wie AIDS und ARC (AIDS Relates Complex). Diese Erkrankungen werden durch Retroviren, insbesondere HIV, besonders HIV-1 und HIV-2, verursacht. Die obigen Verbindungen und Salze sind besonders nützlich zur Milderung der destruktiven cytopathischen Effekte retroviraler Erkrankungen, können aber auch an asymptomatisch HIV-infizierte Patienten verabreicht werden.

Die Erfindung betrifft weiterhin antivirale Präparate in Dosiseinheitsform, die zur oralen oder parenteralen Verabreichung besonders geeignet sind, für die Behandlung der obigen Krankheiten, und die eine therapeutisch wirksame Menge einer der obigen Verbindungen oder Salze enthalten, und gewünschtenfalls eine therapeutisch wirksame Menge einer zweiten anti-HIV-wirksamen Verbindung, insbesondere eines Hemmers der reversen Transcriptase (anti-RT-Mittel) wie ddC, AZT, ddI oder ddA.

Die Erfindung betrifft weiterhin Handelspackungen, die als wirksamen pharmazeutischen Bestandteil eine der obigen Verbindungen A oder B oder ein Salz davon enthalten und gewünschtenfalls eine zweite anti-HIV-wirksame Verbindung zusammen mit Instruktionen für ihre Anwendung.

Die Erfindung betrifft weiterhin ein Verfahren zur Behandlung der oben genannten durch Retroviren verursachten Infektionskrankheiten durch Verabreichung einer Verbindung A oder B oder eines pharmeutisch anwendbaren Salzes davon in einer durch den behandelnden Arzt individuell bestimmten täglichen Dosierung, insbesondere in einer antiviral wirksamen Menge von 0,1 bis 10 mg/kg/Körpergewicht pro Tag in einer oder mehreren Dosen, vorzugsweise in einer Menge von 1 bis 3 mg/kg/Körpergewicht ein oder zwei Mal pro Tag, wobei vorzugsweise der therapeutische Blutspiegel 0,05 bis 10, vorzugsweise 0.1 bis 5 $\mu$M beträgt. Diese Dosierung kann in einer oder mehreren Dosen in verschiedenen Intervallen wie 2, 4, 6, 8, 12 oder 24 Stunden verabreicht werden.

Die obigen Verbindungen weisen anti-retrovirale Aktivität auf, insbesondere eine Hemmwirkung auf das Transaktivatorprotein TAT (anti-TAT Mittel). Wenn ein anti-RT-Mittel wie ddC ebenfalls Verabreicht wird, kann dies im Bereich von 0,05 $\mu$M bis 1,0 $\mu$M, insbesondere 0,005 bis 0,25 mg/kg/Körpergewicht geschehen. Die einleitenden Dosierungsbereiche für die orale Verabreichung können zwischen 0,001 bis 0.25 mg/kg in einer oder mehreren Dosen im Verlauf von Intervallen von 2, 4, 6, 8, 12 Stunden usw. liegen. Gegenwärtig werden 0,01 mg/kg/Körpergewicht ddC alle 8 Stunden gegeben. Bei Verabreichung in der kombinierten Therapie kann das anti-RT Mittel gleichzeitig mit dem anti-TAT Mittel gegeben werden, oder die Dosierung kann gestaffelt erfolgen. Die beiden Mittel können auch in einem Präparat vereinigt werden; die Dosierung jedes Einzelnen kann dann geringer sein. als wenn sie als Einzelsubstanzen verabreicht werden.

Die erfindungsgemässen antiviralen Mittel enthalten mindestens eines der obigen anti-TAT Mittel und gewünschtenfalls andere therapeutische Stoffe, z.B. ein anti-RT Mittel zusammen mit einem oder mehreren pharmazeutisch anwendbaren Trägern. Diese Träger beinhalten solche, die in der galenischen Technologie an sich bekannt sind und für die orale, rektale, nasale, topische, buccale, sublinguale, vaginale oder parenterale (einschliesslich der subkutanen, intramuskulären, intravenösen und intradermalen) Verabreichung geeignet sind. Beispiele erfindungsgemässer Präparate sind Lösungen des (der) Aktivstoffe in Wasser, Kochsalzlösung oder Orangensaft, Kapsel, z.B. Weichgelatinekapseln. Pulver oder Tabletten, die jeweils eine vorbestimmte Menge des aktiven Stoffes enthalten, z.B. als Granulate, Lösungen oder Suspensionen in wässrigen Flüssigkeiten oder als Oel-in-Wasser-Emulsion oder als Wasser-in-Oel-Emulsion. Tabletten können einen oder mehrere Stoffe wie Lactose, mikrokristalline Cellulose, kolloidales Siliciumdioxid, Natriumcroscarmellose, Magnesiumstearat, Stearinsäure und andere Excipientien, Farbstoffe und pharmazeutisch verträgliche Träger enthalten. Formulierungen für die topische Verabreichung umfassen Lutschtabletten, die den aktiven Wirkstoff mit einem Geschmackstoff wie Zucker oder Akaziengummi oder Tragacanth enthalten, Pastillen, die den aktiven Wirkstoff in einer inerten Basis wie Gelatine oder Glycerin oder Sucrose oder Akaziengummi enthalten und Mundwässer, die den aktiven Inhaltsstoff in einem geeigneten flüssigen Träger enthalten. Formulierungen zur rektalen Verabreichung können als Suppositorien mit einer geeigneten Base, wie z.B. Kakaobutter oder einem Salicylat vorliegen. Formulierungen, die für die vaginale Verabreichung geeignet sind, können als Pessare, Tampons, Crèmes, Gele, Pasten, Schäume oder Sprays formuliert sein, die zusätzlich zu dem aktiven Wirkstoff Träger enthalten, wie sie in der galenischen Technik als geeignet bekannt sind. Formulierungen, die für die parenterale Verabreichung geeignet sind, umfassen wässrige oder nicht-wässrige isotonische sterile Injektionslösungen, die Antioxidantien, Puffer, Bakteriostatika und Stoffe enthalten, die die Formulierung isotonisch mit dem Blut des Empfängers machen, und wässrige und nicht-wässrige sterile Suspensionen, die ein Suspendierungsmittel und Verdickungsmittel enthalten. Die Formulierungen können in versiegelten Einfachdosis- oder Mehrfachdosisbehältern vorliegen, beispielsweise Ampullen und Röhrchen, und können in lyophilisiertem Zustand gelagert werden, sodass nur der Zusatz eines sterilen flüssigen Trägers, z.B. Wasser für Injektionszwecke unmittelbar vor Gebrauch nötig ist.

Die Verbindungen der vorliegenden Erfindung wurden auf ihre anti-TAT Aktivität in einer Versuchsanordnung geprüft. die folgende Schritte umfasst:

(a) Die Expression des Gens für die sekretierte alkalische Phosphatase (SeAP) und des Gens für den viralen Transaktivator TAT wird unter die Kontrolle des HIV-Promotors LTR gebracht, der durch die Wirkung des HIV-Transaktivators TAT reguliert wird.

(b) Kultivierte Säugetierzellen werden mit Plasmiden transfiziert, die die Genkonstrukte von (a) enthalten, und die zelluläre Produktion des Transaktivierungsfaktors TAT und SeAP bewirken.

(c) Das zu testende Mittel, hier die Verbindungen A und B, wird zugesetzt, und die Menge des produzierten SeAP wird durch Messen der enzymatischen SeAP Aktivität bestimmt, wobei die Hemmung der SeAP Produktion mit der anti-TAT Hemmaktivität korreliert. Bei dieser Versuchsanordnung korreliert die Hemmung von SeAP positiv mit der anti-TAT Aktivität. Je grösser die Fähigkeit eines Mittels zur Hemmung von SeAP ist, desto grösser ist seine anti-TAT Aktivität.

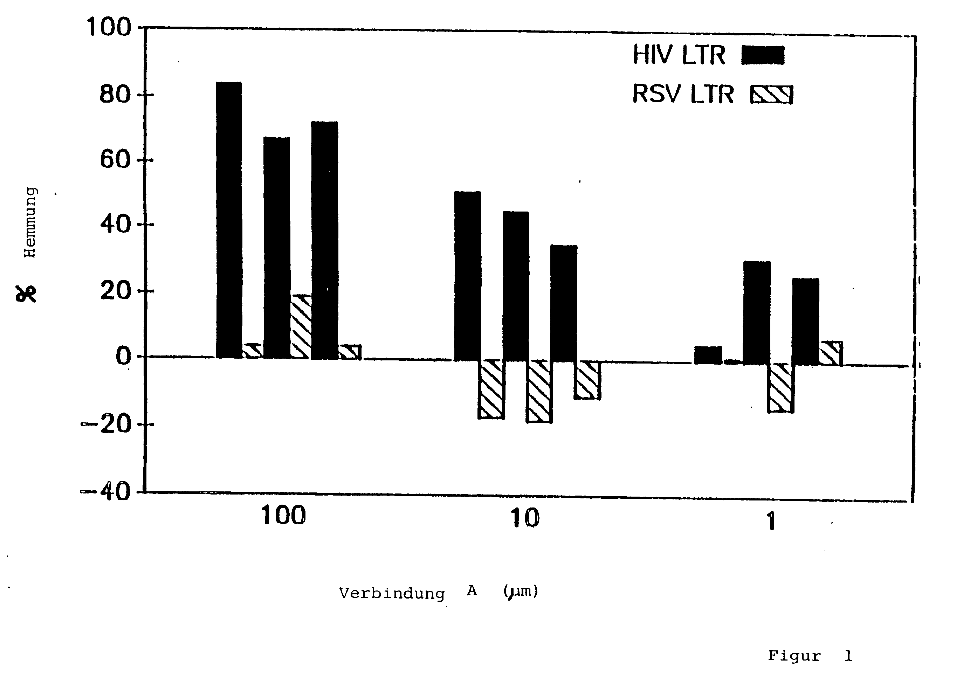

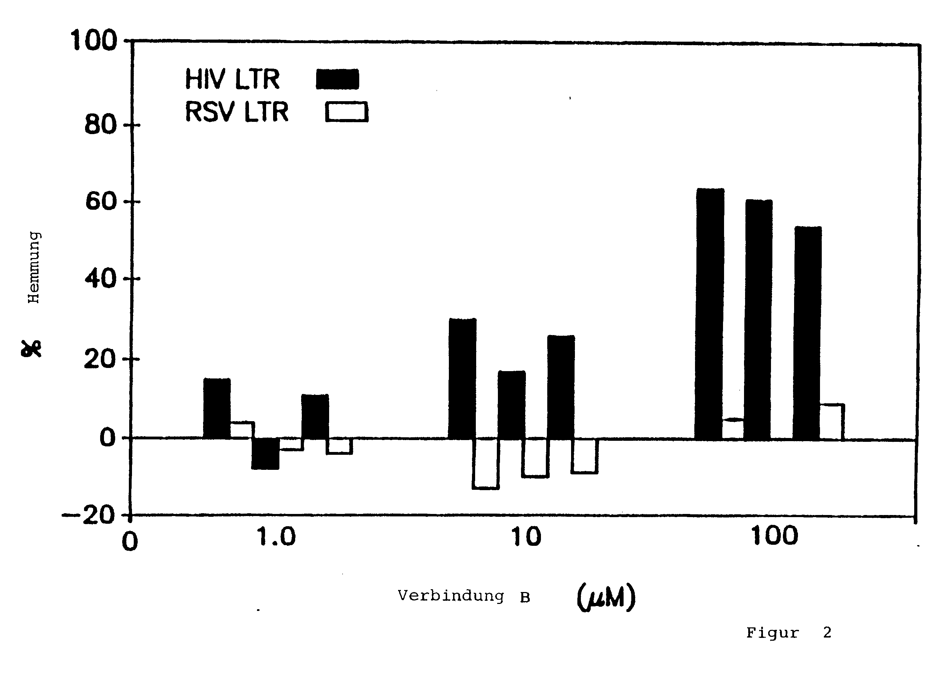

Speziell für die Verbindungen A und B wurde der Versuch, dessen Resultate in Figur 1 und Figur 2 angeführt sind, wie folgt durchgeführt:

24 Stunden nach Transfektion wurden 1, 10 und 100 $\mu$M Testverbindung dem Kulturmedium von COS Zellen zugesetzt, die mit zwei Plasmiden transfiziert waren, nämlich einem, das das Reporter Gen enthielt, das unter Kontrolle von HIV-LTR für SeAP kodiert, und einem anderen mit dem ebenfalls unter Kontrolle von HIV-LTR stehenden TAT Gen. Die alkalische Phosphataseaktivität des Mediums wurde 48 Stunden nach Zusatz der Testverbindungen gemessen. Die Resultate sind in Figur 1 und 2 dargestellt und repräsentieren drei unabhängige Versuchsläufe mit den Testverbindungen. Die anti-TAT Aktivität wird durch die prozentuale Hemmung der unter Kontrolle von HIV-LTR stehenden SeAP Genexpression gemessen. Die Cytotoxizität der Verbindung wurde in einem Parallelversuch geprüft, wobei die SeAP Gen-Expression vom Rous Sarcoma Virus (RSV)-LTR kontrolliert wird, das nicht von TAT beeinflusst wird. Die Resultate sind in Figur 1 und 2 angegeben und zeigen, dass die Verbindungen A und B spezifische Hemmstoffe für TAT sind und keine unspezifische Cytotoxizität aufweisen.

Die Verbindungen A und B wurden auch auf Hemmwirkung von HIV-cytopathischen Effekten und auf die Reduktion Zell-assoziierter viraler Antigene in einem wie folgt durchgeführten Experiment geprüft:

Virusstämme mit hohem Titer (HIV-1 NIT Stamm) wurden in $CD4^+$ CR10 Zellen in RMPI-1640 Medium (Gibco Laboratories), das mit 10% fötalem Kalbsserum und 0,1 mg/ml Gentamicin ergänzt war, gezüchtet. Die gesammelten Medien wurden filtriert und die Virusisolate 100-fach konzentriert und bei -80°C aufbewahrt. Im gleichen Medium gezüchtete $CD4^+$ CEM Zellen wurden 60 Minuten bei 37°C mit verdünnten Viruskulturen bei MOI = 1 inkubiert. Die Zellen wurden mit Phosphat-gepufferter Kochsalzlösung gewaschen und im Medium mit $2 \times 10^5$ Zellen/ml resuspendiert. Verschiedene Mengen der Verbindungen A und B wurden zugesetzt. Drei Tage nach der Infektion wurden die lebenden Zellen durch Trypan-Ausschlussfärbung (Proc. Natl. Acad. Sci. 83, 1986, 1911) gezählt. Zur gleichen Zeit wurden Zellaliquote mit Aceton fixiert und mit Antikörpern von AIDS-Patienten gefärbt, gefolgt von einer zweiten Färbung mit Fluorescein-konjugiertem Ziegen-anti-Human IgG (Cappel). Die mit fluoreszierenden Antikörpern gefärbten Zellen wurden mit einem Fluoreszenzmikroskop gezählt und die Resultate wurden als Prozentsatz der gesamten Anzahl der gezählten Zellen ausgedrückt (J. Med. Virol. 19. 1986. 325).

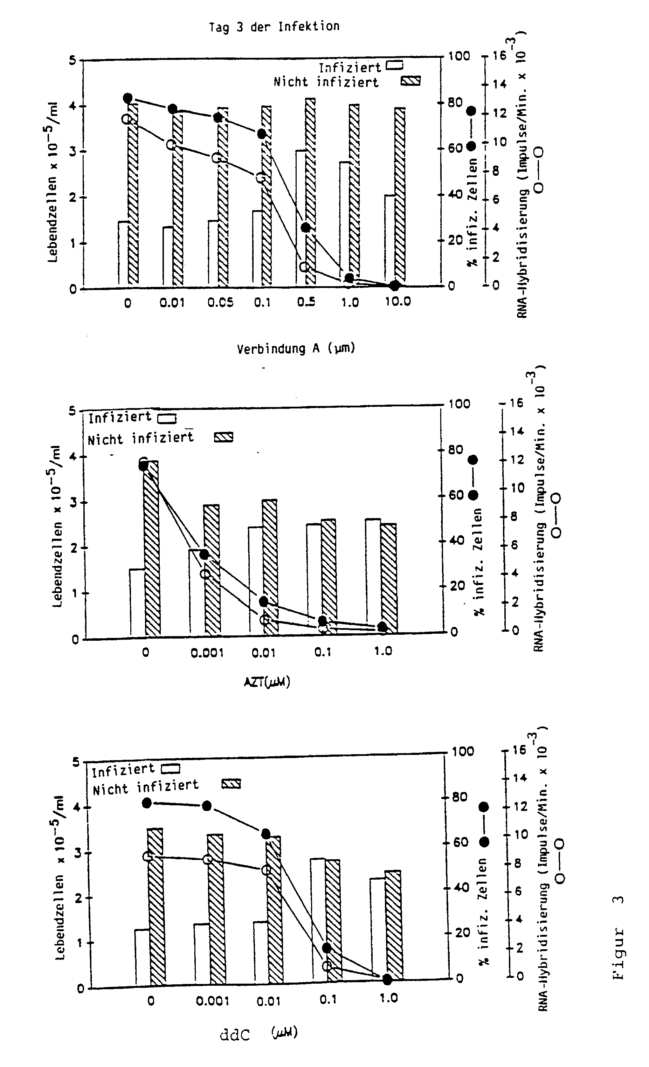

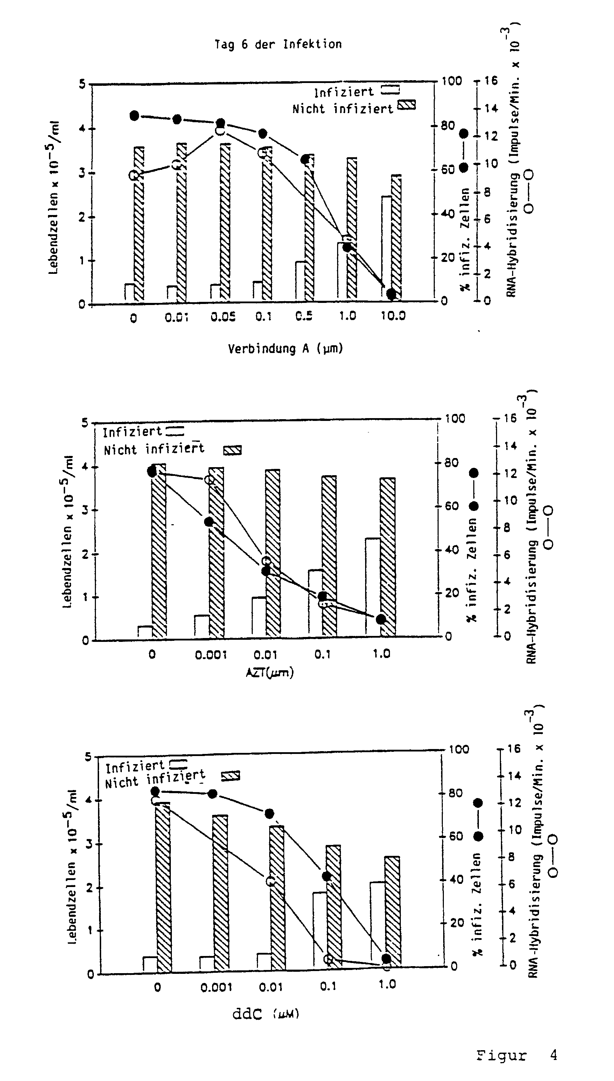

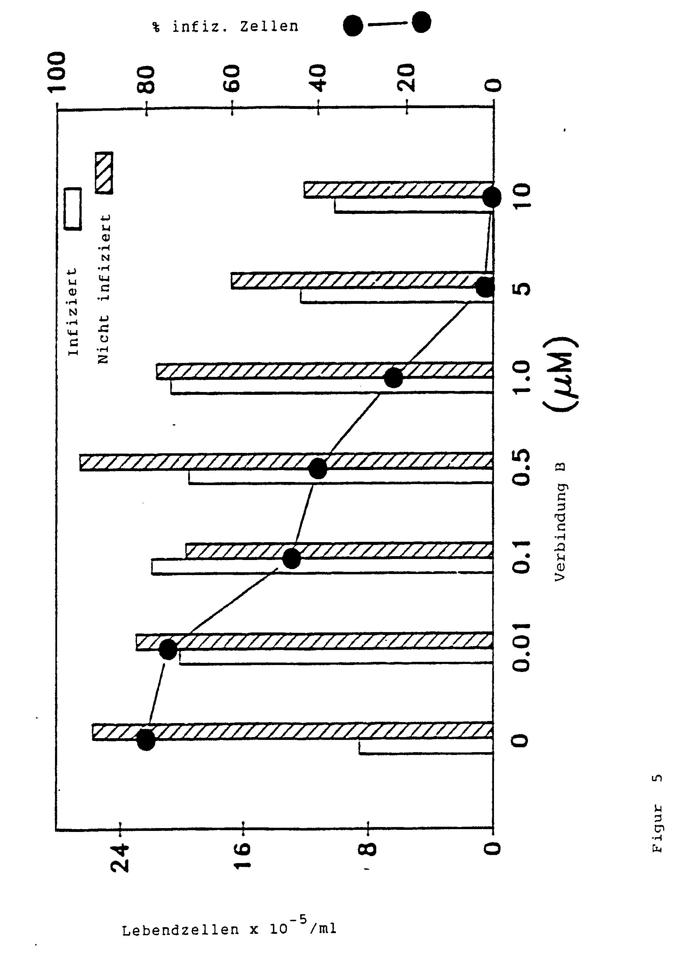

Zur Prüfung auf Cytotoxizität wurden nicht-infizierte CEM Zellen mit den Verbindungen A und B in ähnlichen Konzentrationen geprüft und die Toxizität der Verbindung wurde durch Zählung der Lebendzellen gemessen. Zwei Verbindungen. AZT und ddC mit bekannter Wirksamkeit für die Behandlung von AIDS wurden mit Verbindung A parallel getestet. Um den Effekt einer länger dauernden Behandlung mit Verbindung A zu testen. wurden die mit der Verbindung behandelten Zellen am Tag 3 in die Verbindung enthaltendem Medium wieder ausplattiert und der Test wurde am Tag 6 wiederholt. Die Resultate des Tests sind in den Figuren 3 und 4 für die Verbindung A und in Figur 5 für die Verbindung B angeführt.

Die Verbindung A wurde weiterhin auf antivirale Aktivität im p24 viral antigen capture assay untersucht:

Ein ELISA (Enzyme Linked Immuno-absorbant Assay)-Test wurde zur Prüfung der Verbindung A auf anti-HIV-Aktivität durch Messung der Virusreplikation in CD4[+] T-Lymphocyten verwendet, wobei das virale p24 Antigen, das in den Ueberstand von Gewebekulturen infizierter Zellen freigesetzt wurde, bestimmt wurde.

Virusstämme mit hohem Titer (HIV/HTLV III. RF-Stamm) wurden in H9 Zellen in RPMI-1640 (Flow Laboratories), das mit 10% fötalem Kälberserum, 100 IU/ml Penicillin und 100 $\mu$g/ml Streptomycin ergänzt war, gezüchtet. Die Zelltrümmer wurden abzentrifugiert und der Ueberstand bei -70° C aufbewahrt. Als Wirtszellinie wurde die C8166 CD4[+] T-Zellinie verwendet, die besonders empfindlich auf Infektion mit HIV ist. Die Zellen wurden mit 10 $TCID_{50}$, HIV/HTLV III (RF) bei 37° C 90 Minuten lang inkubiert und dann mit PBS gewaschen. Zellaliquote ($2 \times 10^5$) wurden in 1.5 ml Nährmedium resuspendiert, geeignete Mengen der Verbindung A wurden zugesetzt und es wurde bei 37° C inkubiert. 72 Stunden nach der Infektion wurde der Ueberstand aus den Zellkulturen auf p24 Antigen geprüft. Dieser Antigennachweistest wurde in Ueberständen aus infizierten Zellkulturen ausgeführt. Zu jedem Versuch wurde eine positive Kontrolle vorgenommen, zusammen mit einer Ueberstandskontrolle von nicht-infizierten Zellen. Die Platten wurden mit einem spektrophotometrischen Detektorsystem abgelesen. In zwei getrennten Versuchen wurde für die Verbindung A eine antivirale Aktivität gefunden. wobei etwa 0.1 $\mu$M und 6 $\mu$M Verbindung A 50% Hemmung der viralen Replikation ergaben.

Die folgenden galenischen Präparate. die die oben definierten aktiven Wirkstoffe enthalten, können in an sich bekannter Weise hergestellt werden.

| Tablette: | |
|---|---|
| Inhaltsstoffe | mg/Tablette |
| Verbindung A oder B | 20 mg |
| Lactose | 180 mg |
| vorgelatinisierte Stärke | 15 mg |

| Flüssige Formulierung zur oralen Verabreichung: | |
|---|---|
| Inhaltsstoffe | mg/Formulierung |
| Verbindung A oder B | 20.0 mg |
| Methylparaben | 20.0 mg |
| Sucrose | q.s. |
| Geschmacksstoff | q.s. |
| Citratpuffer | q.s. |
| gereinigtes Wasser q.s. | 5.0 ml |

| Tablette: | | |
|---|---|---|
| Inhaltsstoffe | mg/Tablette | mg/Tablette |
| Verbindung A oder B | 20 mg | 20 mg |
| ddC | 5 mg | |
| Stärke | 40 mg | 40 mg |
| Avicel | 80 mg | 80 mg |
| Lactose | 269 mg | 274 mg |
| Magnesiumstearat | 2 mg | 2 mg |
| | 416 mg | 416 mg |

| Tablette: | |
|---|---|
| Inhaltsstoffe | mg/Tablette |
| Verbindung A oder B | 20 mg |
| ddC | 5 mg |
| Lactose | 175 mg |
| vorgelatinisierte Stärke | 15 mg |
| mikrokristalline Cellulose | 72 mg |
| modifizierte Stärke | 10 mg |
| Magnesiumstearat | 3 mg |
| | 300 mg |

| Weichgelatinekapsel: | |
|---|---|
| Inhaltsstoffe | mg/Kapsel |
| Verbindung A oder B | 20 mg |
| ddC | 0 or 5 mg |
| äthoxylierte Fettsäuren | 500 mg |
| PEG 4000 | 100 mg |
| vegetabile Oele q.s. zu | 1.0 ml |

| Flüssige Formulierung für orale Applikation: | |
|---|---|
| Inhaltsstoffe | mg/ml |
| Verbindung A oder B | 4.0 mg |
| ddC | 1.0 mg |
| Methylparaben | 2.0 mg |
| Propylparaben | 0.2 mg |
| Sucrose | 100.0 q.s. |
| Geschmacksstoff | q.s. |
| Citratpuffer | 5.0 mg |
| gereinigtes Wasser q.s. | 1.0 ml |

| Parenterale Formulierung: | |
|---|---|
| Inhaltsstoffe | mg/ml |
| Verbindung A oder B | 20.0 mg |
| ddC | 5.0 mg |
| Propylenglykol | 20.0 mg |
| Emulphor | 2.0 mg |
| Wasser für Injektion q.s. | 1.0 ml |

**Ansprüche**

1. Verwendung des 7-Chlor-5-(2-pyrryl)-3H-1,4-benzodiazepin-2(1H)-ons, des 2-Glycinamido-5-chlorphenyl-(2-pyrryl)ketons oder pharmazeutisch anwendbare Salze davon zur Herstellung von pharmazeutischen Präparaten zur Behandlung oder Prophylaxe von durch Retroviren verursachten Infektionskrankheiten, besonders zur Linderung destruktiver cytophatischer Effekte retroviraler Erkrankungen, insbesondere solche, die durch Retrovirus HIV, insbesondere HIV-1, verursacht werden.

2. Antivirale Präparate in Dosiseinheitsform, die für die orale und parenterale Verabreichung zur Behandlung oder Prophylaxe gemäss Anspruch 1 geeignet sind, enthaltend eine therapeutisch wirksame Menge einer Verbindung gemäss Anspruch 1 und gewünschtenfalls eine therapeutisch wirksame Menge einer zweiten anti-HIV Verbindung, insbesondere eines Hemmers der reversen Transcriptase, insbesondere ddC.

3. Handelspackung, enthaltend als aktiven pharmazeutischen Wirkstoff eine Verbindung gemäss Anspruch 1 und. gewünschtenfalls, eine zweite anti-HIV wirksame Verbindung gemäss Anspruch 2 zusammen mit Instruktionen zur Anwendung gemäss Anspruch 1.

Figur 1

EP 0 429 868 A2

Figur 2

Tag 3 der Infektion

Verbindung A (μm)

AZT(μM)

ddC  (μM)

Figur  3

Figur 4

Figur 5